# FASCICULE DE BREVET EUROPEEN

(11) **EP 1 080 096 B1**
(45) Date de publication et mention de la délivrance du brevet: **09.04.2003**
(21) Numéro de dépôt: 99918056.5
(22) Date de dépôt: 11.05.1999
(51) Int. Cl.: C07F 9/655, C07F 15/00, C07C 45/50, C08G 67/02, C07C 253/30

(54) **NOUVELLES FURYLPHOSPHINES ET COMPLEXES ORGANOMETALLIQUES LES COMPRENANT**
NEUE FURYLPHOSPHANE UND SIE ENTHALTENDE ORGANOMETALL-KOMPLEXE
NOVEL FURYLPHOSPHINES AND ORGANOMETALLIC COMPLEXES CONTAINING THEM

(30) Priorité: 20.05.1998 FR 9806559
(43) Date de publication de la demande: 07.03.2001
(73) Titulaire: Rhodia Polyamide Intermediates, 69192 Saint-Fons (FR)
(72) Inventeur: MATHEY, François, F-75004 Paris (FR); SAVIGNAC, Philippe, F-91190 Gif-sur-Yvette (FR); EYMERY, Frédéric, F-94140 Alfortville (FR); BURATTIN, Paolo, F-69002 Lyon (FR)
(74) Mandataire: Esson, Jean-Pierre
(86) Numéro de dépôt international: FR9901129
(87) Numéro de publication internationale: WO99060003

(56) Documents cités:
- EP-A- 0 305 012
- FR-A- 2 314 910
- V. FARINA: "Large rate accelarations in the Stille reaction with tri-2-furylphosphine and trifurylarsine as palladium ligands: mechanistic and synthetic implications" JOURNAL OF THE AMERICAN CHEMICAL SOCIETY., vol. 113, no. 25, 1991, pages 9585-9595, XP002090428 DC US cité dans la demande

## Description

La présente invention concerne de nouvelles furylphosphines hydrosolubles.

Elle concerne également des complexes organométalliques comprenant ces furylphosphines et l'utilisation de tels complexes.

Elle a trait aussi au procédé de préparation des furylphosphines hydrosolubles.

Des trifurylphosphines non substuées sont décrites dans un article de V. Farina et B. Krishnan publié dans Journal of American Chemical Society, 1991, 113, n°25 pages 9585-9595. Des systèmes comprenant ces trifurylphosphines présentent selon cet article une activité catalytique remarquable.

Différents systèmes catalytiques notamment utilisés dans la transformation d'oléfines ont été proposés. Ainsi, le brevet FR2314910 propose d'utiliser une triarylphosphines hydroslubles dans une réaction d'hydroformylation d'oléfines. Le brevet EP0305012 décrit l'utilisation de diphosphines en association avec du palladium dans la synthèse de polycétones.

L'art antérieur ne décrit pas de furylphosphines hydrosolubles telles que celles faisant l'objet de la présente invention.

Les nouvelles furylphosphines hydrosolubles répondent à la formule générale (I) : 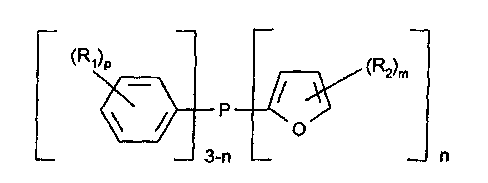 dans laquelle :
- n représente un nombre entier de 1 à 3
- au moins un radical R₂ représente un groupe hydrophile choisi dans le groupe comprenant
   - SO₂M, -SO₃M, -CO₂M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 12 atomes de carbone, les autres cations dérivés de métaux comprenant les dérivés métalliques obtenus avec des acides furylsulfiniques, des acides furylcarboxyliques, des acides furylsulfoniques ou des acides furylphosphoniques et qui sont solubles dans l'eau,
   - N(R)₃X dans la formule duquel les symboles R, identiques ou différents, représentent un atome d'hydrogène ou un radical alkyle ayant de 1 à 12 atomes de carbone, X représente un anion organique ou minéral
   - OH
- R₁ représente un groupe hydrophile tel que défini pour R₂ ou un groupe alkyle ou alcoxy ayant 1 à 12 atomes de carbone, un atome d'halogène, un groupe nitrile ou un groupe halogénoalkyle ayant 1 à 12 atomes de carbone,
- m représente 1 ou 2
- p représente un nombre entier de 0 à 3
- lorque m est égal à 2, un radical R₂ peut représenter un groupe alkyle ou alcoxy ayant 1 à 12 atomes de carbone, un atome d'halogène, un groupe nitrite ou un groupe halogénoalkyle ayant 1 à 12 atomes de carbone.

Par le terme hydrosoluble ou l'expression soluble dans l'eau, on entend dans le présent texte un composé soluble à au moins 0,01 g par litre d'eau.

Les furylphosphines hydrosolubles de l'invention sont le plus souvent des composés de formule générale (I) dans laquelle :
- n représente un nombre entier de 1 à 3,
- R₂ représente un groupe hydrophile tel que -SO₂M, -SO₃M, -CO₂M, -PO₃M, M représentant un reste cationique minéral ou organique choisi parmi le proton, les cations dérivés des métaux alcalins ou alcalino-terreux, les cations ammonium - N(R)₄ dans la formule desquels les symboles R, identiques ou différents, représentent un atome d'hydrogène ou un radical alkyle ayant de 1 à 4 atomes de carbone, les autres cations dérivés de métaux comprenant les dérivés métalliques obtenus avec des acides furylsulfiniques, des acides furylcarboxyliques, des acides furylsulfoniques ou des acides furylphosphoniques et qui sont solubles dans l'eau,
- m représente 1 ou 2,
- R₁ représente un groupe hydrophile tel que défini pour R₂ , ou un substituant -N(R)₃ X dans la formule duquel les symboles R, identiques ou différents, représentent un atome d'hydrogène ou un radical alkyle ayant de 1 à 4 atomes de carbone et X représente un anion organique ou minéral, un substituant -OH, un substituant alkyle ou alcoxy ayant 1 à 4 atomes de carbone, un atome d'halogène, un groupe nitrile ou un groupe trifluoroalkyle,
- p représente un nombre entier de 0 à 2.

La présente invention a également pour objet la préparation des nouvelles furylphosphines hydrosolubles. Cette préparation est généralement effectuée à partir de d'un précurseur tel que diphényl-furylphosphine ou phényl-difurylphosphine ou trifurylphosphine non substituée par des groupes hydrophiles. Elle consiste à introduire sur les cycles furyle les groupes hydrophiles R₂ et éventuellement sur les cycles phényle les groupes R₁.

Par exemple, on peut ainsi préparer de manière générale les furylphosphines de formule (I) par condensation d'un composé organolithien, dont la partie organique correspond à un composé de formule générale (I) ne comportant pas de substituants R₂ et qui est lié à l'atome de lithium par son ou ses cycles furyle, sur le centre électrophile d'un précurseur de R₂ comme par exemple le dioxyde de soufre, le dioxyde de carbone, les chlorophosphates d'alkyle, les sulfonates de pyridine ou les sulfonates de trialkylamine.

Le composé organolithien est lui-même obtenu par action d'une base lithiée (par exemple butyllithium) sur la furylphosphine précurseur.

Pour la préparation des furylphosphines précurseurs, on peut se référer par exemple à l'article de A.J. Zapata et A.C. Rondon dans Org. Prep. Proced. Int. 27, 5 (1995), pages 567 et suivantes.

Les furylphosphines hydrosolubles permettent de préparer des complexes organométalliques qui comprennent au moins une furylphosphine hydrosoluble de formule (I) et au moins un métal.

Les métaux qui peuvent être complexés par les furylphosphines hydrosolubles sont de manière générale tous les métaux de transition des groupes 1b, 2b, 3b, 4b, 5b, 6b, 7b et 8 de la Classification périodique des éléments, telle que publiée dans "Handbook of Chemistry and Physics, 51st Edition (1970-1971)" de The Chemical Rubber Company.

Parmi ces métaux, on peut citer plus particulièrement les métaux pouvant être utilisés comme catalyseurs de réactions chimiques. Ainsi on peut mentionner à titre d'exemples le nickel, le cobalt, le fer, le ruthénium, le rhodium, le palladium, l'osmium, l'iridium, le platine, le cuivre, l'argent, l'or, le zinc, le cadmium, le mercure.

La préparation des complexes organométalliques comprenant les furylphosphines hydrosolubles peut être effectuée en mettant en contact une solution d'un composé du métal choisi avec une solution aqueuse de la furylphosphine hydrosoluble de formule (I).

Le composé du métal peut être dissous dans l'eau ou dans un solvant organique, ledit solvant organique pouvant lui-même être miscible ou non-miscible à l'eau.

Le métal peut se trouver dans le composé mis en oeuvre, soit au degré d'oxydation qu'il aura dans le complexe organométallique, soit à un degré d'oxydation supérieur.

A titre d'exemples, on peut indiquer que dans les complexes organométalliques de l'invention, le rhodium est au degré d'oxydation (I), le ruthénium au degré d'oxydation (II), le platine au degré d'oxydation (I), le palladium au degré d'oxydation (II), l'osmium au degré d'oxydation (0), l'iridium au degré d'oxydation (0), le nickel au degré d'oxydation (0).

Si lors de la préparation du complexe organométallique, le métal est mis en oeuvre à un degré d'oxydation plus élevé, il devra être réduit in situ.

Les complexes organométalliques comprenant les furylphosphines hydrosolubles de formule (I) peuvent être utilisés comme catalyseurs de réactions chimiques.

En catalyse biphasique aqueuse, les furylphosphines hydrosolubles bénéficient de la synergie entre l'hydrophilie naturelle du cycle furyle et celle du ou des radicaux R₂. Elles atteignent des valeurs de solubilité dans l'eau très élevées.

Comme réactions chimiques pouvant être catalysées par des complexes organométalliques comprenant les furylphosphines hydrosolubles de formule (I), on peut citer par exemple l'hydroformylation et l'hydrocarbonylation des oléfines en présence de complexes du rhodium, l'hydrogénation des oléfines, des aldéhydes, des acides, des énamides et des composés nitroaromatiques en présence de complexes du ruthénium, du rhodium, du platine ou du palladium, la télomérisation des diènes, l'isomérisation des oléfines, la dimérisation de l'éthylène ou de l'acrylonitrile, l'hydrocyanation des oléfines en présence de complexes du nickel, la synthèse du furanne en présence de complexes du ruthénium, la métathèse des oléfines en présence de complexes du ruthénium, la polymérisation des acrylates en présence de complexes du nickel, ou les réactions de couplage carbone-carbone comme par exemple la réaction de HECK, ou de SUZUKI en présence de complexes du nickel ou du palladium.

Les composés de l'invention sont notamment utiles pour la réaction d'isomérisation des nitriles obtenus par hydrocyanation d'un diène, et plus particulièrement l'isomérisation du 2-méthyl 3-butène en 3-pentène nitrite. Cette réaction a une grande importance industrielle dans le procédé de fabrication de l'adiponitrile. Ce dernier est un grand intermédiaire de synthèse pour notamment la fabrication des monomères de polyamides tels que le caprolactame ou l'hexaméthylène diamine.

Les exemples qui suivent illustrent l'invention.

### EXEMPLES 1 A 4 : synthèse de furylphosphines hydrosolubles de formule (I)

### EXEMPLE 1 : 2-(diphénylphosphino)-furyl-5-carboxylate de sodium

Dans un ballon tétracol de 500 ml, préalablement purgé à l'azote et surmonté d'une ampoule isobare, d'un réfrigérant muni d'un bulleur, d'une agitation mécanique et d'un thermomètre, on charge 30,3 ml (47 mmol) de n-butyllithium (dosé à 1,55 M), puis on incorpore rapidement 6,4 ml (42 mmol) de tétraméthyléthylènediamine (TMEDA) dans 50 ml d'éther éthylique anhydre dégazé à l'azote. On coule goutte à goutte 10,65 g (42,3 mmol) de diphénylfurylphosphine préalablement dissoute dans 50 ml d'éther éthylique anhydre dégazé à l'azote, en laissant l'exothermie se développer jusqu'à 35°C. Le mélange se teinte en orange.

Après 15 min d'agitation sous courant d'azote, on contrôle en RMN ³¹P la formation de l'anion. Cette formation est complète 15 min après la fin d'addition de la phosphine.

On adapte alors à la place du réfrigérant une canule qui permet de transférer la solution goutte à goutte dans un vase Dewar contenant du dioxyde de carbone solide immergé dans environ 200 ml d'éther éthylique anhydre dégazé à l'azote. L'anion se condense immédiatement pour donner un précipité blanc. Après une nuit à l'air libre, le solide blanc en suspension dans l'éther est repris par 100 ml d'une solution saturée de NaHCO₃ et l'ensemble est extrait. La phase éthérée est encore extraite par deux fois 30 ml d'eau dégazée. Les phases aqueuses sont réunies, acidifiées à froid par de petites portions de HCl 12 N jusqu'à pH neutre. On extrait cette phase aqueuse par trois fois 50 ml de dichlorométhane. Les phases organiques sont réunies et on leur ajoute 1,49 g de soude dissoute dans quelques ml d'eau. Après évaporation des solvants, on obtient une huile brun-jaune visqueuse qui se transforme en fine poudre blanche après plusieurs triturations et lavages dans de l'éther éthylique anhydre et après séchage à 90°C sous vide. On récupère 11,44 g du produit recherché (36 mmol), soit un rendement de 85 %.

Il est caractérisé par :
- RMN ³¹P (D₂O) : δ = 25,1
- RMN ¹H (D₂O) : δ = 6,20 (d, 1H, ³J_{HH} = 3,2 , C₃H) ; δ = 6,81 (m, 1H, C₄H) ; δ = 7,06 (m, 10H, Ar)
- RMN ¹³C (D₂O) : δ = 117,8 (s, C₃H) ; δ = 124,9 (m, C₄H) ; δ = 131,1 (d, ³J_{CP} = 7,0, C_{méta}) ; δ = 131,7(s, Cₚₐᵣₐ) ; δ = 135,5 (d, ²J_{CP} = 19,7, Cₒᵣₜₕₒ) ; δ = 136,8 (d, ¹J_{CP} = 4,4, Cᵢₚₛₒ) ; δ = 156,4 (s, COO) ; δ = 156,9 (d, ¹J_{CP} = 13,7, C₅H) ; δ = 168,1 (s, C₂H).

La solubilité dans l'eau de cette furylphosphine est de 250 g/l à 23°C.

### EXEMPLE 2 : 2-(diphénylphosphino)-furyl-5-phosphonate de disodium

Dans un ballon tétracol de 500 ml, préalablement purgé à l'azote et surmonté d'une ampoule isobare, d'un réfrigérant muni d'un bulleur, d'une agitation mécanique et d'un thermomètre, on charge 33,8 ml (52 mmol) de n-butyllithium (dosé à 1,55 M), puis on incorpore rapidement 6,4 ml (42 mmol) de TMEDA dans 50 ml d'éther éthylique anhydre dégazé à l'azote. On coule goutte à goutte 12,32 g (48,9 mmol) de diphénylfurylphosphine préalablement dissoute dans 40 ml d'éther éthylique anhydre dégazé à l'azote, en laissant l'exothermie se développer jusqu'à 35°C. Le mélange se teinte en orange.

Après 15 min d'agitation sous courant d'azote, on contrôle en RMN ³¹P la formation de l'anion. Cette formation est complète 15 min après la fin d'addition de la phosphine.

On refroidit le mélange réactionnel à -70°C et on adapte à la place du réfrigérant une canule. On transfère la solution goutte à goutte dans un second réacteur tétracol équipé du même appareillage que le premier, refroidi à -60°C et contenant 7,3 ml (49 mmol) de diéthylchlorophosphate dilué dans 20 ml d'éther éthylique anhydre. On laisse le milieu revenir à température ambiante avant d'hydrolyser in situ par 100 ml d'une solution aqueuse de chlorure d'ammonium. On extrait alors par trois fois 50 ml d'éther éthylique. Les phases organiques sont réunies et on les sèche sur sulfate de Mg. On filtre et on évapore pour obtenir 20 g d'une huile brun-jaune. Celle-ci est chauffée à 100°C sous 10⁻² mm de mercure (1,3 Pa) dans un four à boules afin d'éliminer les phosphates. On récupère 17,2 g d'une huile visqueuse brun-jaune qui est ensuite chromatographiée sur 10 cm de gel de silice avec un mélange 50/50 acétate d'éthyle/hexane comme éluant. Après évaporation des solvants, on recueille 14,2 g d'une huile colorée que l'on place pendant une nuit en solution dans 60 ml de dichlorométhane, sous agitation magnétique, en présence de 10 ml (74 mmol) de bromotriméthylsilane. La réaction est exothermique et l'on observe une décoloration du milieu. Après évaporation des solvants, le produit est repris dans 60 ml d'acétone et 2 ml d'eau distillée. Après 2 h 30 les solvants sont évaporés et le produit est repris dans le minimum de méthanol. A froid on incorpore sous agitation 3,04 g de soude en solution aqueuse à 40 %. Il se forme un précipité blanc. Les solvants sont évaporés et le produit est lavé à l'acétone, filtré et séché à 100°C sous vide. On récupère le produit recherché avec un rendement de 54 %.

Il est caractérisé par :
- RMN ³¹P (D₂O) : δ = 28,2 ; d = 0,8 (P=O)
- RMN ¹H (D₂O) : δ = 6,72 (m, 2H, C₃H, C₄H) ; δ = 7,41 (m, 10H, Ar)
- RMN ¹³C (D₂O) : δ = 117,9 (dd, ⁴J_{CP} = 4,67, ³J_{CP} = 20,0, C₃H) ; δ = 124,6 (dd, J_{CP} = 3,5, J_{CP} = 21,0, C₄H) ; δ = 131,6 (d, ³J_{CP} = 7,5, C_{méta}) ; δ = 132,2(s, Cₚₐᵣₐ) ; δ = 135,8 (d, ²J_{CP} = 19,2, Cₒᵣₜₕₒ) ; δ = 137,8 (s, Cᵢₚₛₒ) ; δ = 155,4 (dd, J_{CP} = 7,4, J_{CP} = 10,4, C₅H) ; δ = 165,1 (d, ¹J_{CP} = 199,0, C₂H).

La solubilité dans l'eau de cette furylphosphine est de 680 g/l à 20°C.

### EXEMPLE 3 : 2-2'-phénylphosphinediyl-bis(furyl-5-phosphonate de disodium)

Dans un ballon tétracol de 500 ml, préalablement purgé à l'azote et surmonté d'une ampoule isobare, d'un réfrigérant muni d'un bulleur, d'une agitation mécanique et d'un thermomètre, on charge 42 ml (65 mmol) de n-butyllithium (dosé à 1,55 M), puis on incorpore rapidement 9,15 ml (60 mmol) de TMEDA dans 50 ml d'éther éthylique anhydre dégazé à l'azote. On coule goutte à goutte 4,41 ml (60,0 mmol) de furanne préalablement dilué dans 40 ml d'éther éthylique anhydre dégazé à l'azote, en laissant l'exothermie se développer jusqu'à 35°C pendant 30 min. Le mélange se teinte en orange.

On refroidit à -40°C pour couler goutte à goutte 4,2 ml (30 mmol) de dichlorophénylphosphine diluée dans 20 ml d'éther, Après contrôle en RMN ³¹P de la formation du produit, on remonte à température ambiante pour incorporer 9,15 ml (60 mmol) de TMEDA dans 50 ml d'éther éthylique, puis on coule rapidement 42 ml (65 mmol) de n-butyllithium (dosé à 1,55 M). Après 15 min d'agitation sous courant d'azote, on contrôle en RMN ³¹P la formation de l'anion. Cette formation est complète 15 min après la fin d'addition de la phosphine.

On refroidit le mélange réactionnel à -70°C et on adapte à la place du réfrigérant une canule. On transfère la solution goutte à goutte dans un second réacteur tétracol équipé du même appareillage que le premier, refroidi à -60°C et contenant 8,95 ml (60 mmol) de diéthylchlorophosphate dilué dans 20 ml d'éther éthylique anhydre. On laisse le milieu revenir à température ambiante avant d'hydrolyser in situ par 100 ml d'une solution aqueuse de chlorure d'ammonium. On extrait alors par trois fois 50 ml d'éther éthylique. Les phases organiques sont réunies et on les sèche sur sulfate de Mg. On filtre et on évapore pour obtenir 20 g d'une huile brun-rouge. Celle-ci est chauffée à 100°C sous 10⁻² mm de mercure (1,3 Pa) dans un four à boules afin d'éliminer les phosphates. Le produit est ensuite chromatographié sur 10 cm de gel de silice avec de l'acétate d'éthyle comme éluant. On élimine ainsi de la difurylphénylphosphine et du composé monophosphorylé. Après évaporation des solvants, on recueille 3,5 g de produit pur (rendement : 23 %). L'ensemble de la réaction est reproduit une seconde fois afin d'obtenir suffisamment de produit pour la suite de la synthèse.

On place pendant une nuit 8,55 g (16,6 mmol) de composé diphosphorylé en solution dans 50 ml de dichlorométhane, sous agitation magnétique, en présence de 9,43 ml (70 mmol) de bromotriméthylsilane. La réaction est exothermique et l'on observe une décoloration du milieu. Après évaporation des solvants, le produit est repris dans 50 ml d'acétone et 2 ml d'eau distillée. Après 2 h 30, les solvants sont évaporés et le produit est repris dans le minimum de méthanol. A froid on incorpore sous agitation 2,8 g de soude en solution aqueuse à 40 %. Le milieu fonce du jaune au brun. Les solvants sont évaporés et le solide orange résultant est trituré et lavé à l'acétone, filtré et séché à 100°C sous vide. On récupère 6,7 g du produit recherché de couleur blanchâtre avec un rendement de 82 % (par rapport au composé diphosphorylé).

Il est caractérisé par :
- RMN ³¹P (D₂O) : δ = 49,5 ; d = 0,0 (P=O)
- RMN ¹H (D₂O) : δ = 6,67 (m, 2H, C₄H) ; δ = 6,80 (m, 2H, C₃H) ; δ = 7,41 (m, 5H, Ar)
- RMN ¹³C (D₂O) : δ = 117,7 (d, ³J_{CP} = 20,4, C₃H) ; δ = 123,9 (dd, ³J_{CPO} = 9,2, ²J_{CP} = 18,3, C₄H) ; δ = 131,4 (d, ³J_{CP} = 7,0, C_{méta}) ; δ = 131,9 (s, Cₚₐᵣₐ) ; δ = 134,7 (d, ²J_{CP} = 19,0, Cₒᵣₜₕₒ) ; δ = 136,9 (s, Cᵢₚₛₒ) ; δ = 153,5 (d, ¹J_{CP} = 7,3, J_{CP} = 10,4, C₅H) δ = 164,5 (d, ¹J_{CPO} = 199,5, C₂H).

La solubilité dans l'eau de cette furylphosphine est de 1140 g/l à 23°C.

### EXEMPLE 4: 2-(diphénylphosphino)-furyl-5-sulfinate de sodium

Dans un ballon tétracol de 500 ml, préalablement purgé à l'azote et surmonté d'une ampoule isobare, d'un réfrigérant muni de deux bulleurs (eau et huile de silicone), d'une agitation mécanique et d'un thermomètre, on charge 26,3 ml (40 mmol) de n-butyllithium (dosé à 1,52 M), puis on coule goutte à goutte 10,65 g (42,3 mmol) de diphénylfurylphosphine préalablement dissoute dans 50 ml d'éther éthylique anhydre dégazé à l'azote, en laissant l'exothermie se développer jusqu'à 30°C. Le mélange se teinte en jaune.

Après 45 min d'agitation sous courant d'azote, on contrôle en RMN ³¹P la formation de l'anion. On refroidit le mélange réactionnel à -60°C et on remplace le courant d'azote par un courant de dioxyde de soufre. Un précipité blanc apparaît, puis le milieu se colore en jaune, ensuite en brun. La réaction est exothermique. On rétablit ensuite un fort courant d'azote pour dégazer le réacteur et on laisse le milieu revenir lentement à température ambiante. On rajoute ensuite in situ 100 ml d'eau dégazée et on laisse sous agitation pendant une heure. On extrait trois fois à l'eau. Les phases aqueuses sont réunies, refroidies par un bain de glace et acidifiées par 10 ml de HCI 3N en présence de 100 ml de dichlorométhane. La couleur jaune disparaît. On extrait alors rapidement trois fois par du dichlorométhane. Les phases organiques sont réunies et on incorpore à froid sous agitation magnétique 1,2 g de soude. On évapore les solvants sous vide en limitant la température à 40°C au maximum. On obtient 6,86 g (20,3 mmol) du produit recherché (solide blanc) ; le rendement est de 51 %.

Le produit est caractérisé par :
- RMN ³¹P (D₂O) : δ = 26,7
- RMN ¹H (D₂O) : δ = 6,40 (m, 1H, C₃H) ; δ = 6,53 (m, 1H, C₄H) ; δ = 7,06 (m, 10H, Ar)
- RMN ¹³C (D₂O) : δ = 111,6 (s, C₃H) ; δ = 124,8 (m, C₄H) ; δ = 131,1 (d, ³J_{CP} = 7,0, C_{méta}) ; δ = 131,7 (s, Cₚₐᵣₐ) ; δ = 135,5 (d, ²J_{CP} = 19,4, Cₒᵣₜₕₒ) ; δ = 136,8 (d, ¹J_{CP} = 2,9 , Cᵢₚₛₒ) ; δ = 156,5 (d, J_{CP} = 16,5, C₅H) ; δ = 170,1 (s, C₂H).

La solubilité dans l'eau de cette furylphosphine est de 205 g/l à 21°C.

### EXEMPLES 5 A 13 : préparation de complexes métalliques de furylphosphines hydrosolubles de formule (I) et utilisation de ces complexes comme catalyseurs d'hydrogénation

On opère dans un autoclave de 50 ml, dont les parois sont revêtues de Teflon®, équipé d'un barreau magnétique, d'une valve de sûreté, d'un manomètre, d'une vanne d'arrivée de gaz et d'une vanne d'introduction de liquide.

On prépare tout d'abord les différents complexes du rhodium (exemples 5 à 8), qui seront ensuite utilisés comme catalyseurs pour l'hydrogénation de l'acide Z-α-acétamidocinnamique (exemples 9 à 13).

La préparation des complexes du rhodium consiste à agiter pendant 10 min sous atmosphère d'argon une solution dans 2 ml d'eau de 0,05 mmol de l'une des furylphosphines hydrosolubles préparées dans les exemples 1 à 4, mélangée à une solution dans 1 ml d'acétone de 0,025 mmol de [Rh(COD)₂]⁺, PF₆⁻ (COD = cyclooctadiène). Après le changement de coloration du milieu de l'orange au jaune, on contrôle en RMN ³¹P.

Le tableau 1 ci-après rassemble les éléments de caractérisation des complexes du Rh préparés.

**Tableau 1**

| **Exemples** | **Furyl** **phosphine** | **Aspect** | **δ**^{**31**}**P (en ppm)** | **J**_{**Rh-P**} **(Hz)** |
|---|---|---|---|---|
| Ex 5 | Exemple 1 | jaune vif limpide | 14,6 (d) | 144 |
| Ex 6 | Exemple 4 | jaune vif limpide | 15,0 (d) | 148 |
| Ex 7 | Exemple 2 | jaune vif limpide | 12,0 (d, P_{III}) -1,7 (s, P=O) | 148 |
| Ex 8 | Exemple 3 | jaune vif limpide | -1,0 (s, P=O) -4,0 (d,P_{III}) | 150 |

On introduit dans l'autoclave à l'aide d'une seringue le complexe préparé précédemment, puis une solution de 0,410 g (2 mmol) d'acide Z-α-acétamidocinnamique dans 30 ml de méthanol et d'eau (en proportions volumiques de 1/1 ou 1/2)

Le mélange reste homogène.

On établit une pression de 3 à 4 bar (0,3 à 0,4 MPa) d'hydrogène et on agite pendant un temps variable (voir tableau 2 ci-après) selon la nature de la furylphosphine et l'absorption de gaz. En fin d'essai, après dégazage, le mélange réactionnel est évaporé à sec et analysé par RMN ¹H dans le diméthylsulfoxyde d6.

Le tableau 2 ci-après rassemble les caractéristiques des différents exemples (référence du complexe, rapport pondéral complexe organométallique/substrat à hydrogéner en %, pression H₂, durée) et les résultats obtenus (taux de conversion du substrat).

**Tableau 2**

| **Exemples** | **Complexe** **(référence** **exemple)** | **Complexe** **% en** **poids** | **Rapport** **méthanol** **/eau** | **Pression** **H**_{**2**} | **Durée** | **Taux** **conversion** |
|---|---|---|---|---|---|---|
| Ex 9 | Exemple 5 | 1,25 | 1/2 | 4,0 bar | 3h 30 | 66% |
| Ex 10 | Exemple 5 | 1,25 | 1/1 | 3,0 bar | 4h 30 | 95 % |
| Ex 11 | Exemple 7 | 1,25 | 1/1 | 3,1 bar | 2h | 100 % |
| Ex 12 | Exemple 6 | 0,5 | 1/1 | 3,0 bar | 18h 30 | 8 % |
| Ex 13 | Exemple 8 | 1,25 | 1/1 | 3,1 bar | 2h | 100 % |

### EXEMPLES 14 A 16 : préparation de complexes métalliques de furylphosphines hydrosolubles de formule (I) et utilisation desdits complexes pour l'hydroformylation du styrène

### Préparation des complexes

On préforme le complexe organométallique en additionnant goutte à goutte la furylphosphine de formule (I) dissoute dans 1 ml d'eau au précurseur métallique [Rh(CO)₂Cl]₂ ou [Rh(CO)₂acac], l'abréviation "acac" signifiant "acétylacétonate", en solution soit dans 1 ml d'acétone (variante (a) dans le tableau 3 ci-après) ce qui conduit à un milieu résultant homogène, soit dans 1 ml de toluène (variante (b) dans le tableau 3 ci-après) ce qui conduit à un milieu résultant hétérogène (biphasique).

Lorsque l'on opère en milieu hétérogène, on observe un transfert de la coloration jaune de la phase organique vers la phase aqueuse et la solution obtenue est limpide.

Lorsque l'on opère en milieu homogène, une partie du métal reprécipite sous forme d'aiguilles vert-métal et la couleur du milieu est jaunâtre, voire noire.

L'utilisation comme précurseur du dimère [Rh(CO)₂Cl]₂ nécessite l'ajout de triéthylamine (0,1 ml).

Les complexes ainsi préparés ont été analysés par RMN ³¹P à température ambiante. Ils conduisent tous à un spectre présentant un pic large (caractéristique d'une complexation avec échange rapide de ligands) d'un déplacement chimique δ = -10 à + 20 ppm en fonction de la phosphine utilisée.

### Hydroformylation du styrène

On purge à l'argon un autoclave de 250 ml, équipé d'un barreau magnétique, d'une valve de sûreté, d'un manomètre et de deux vannes d'arrivée de gaz. Sous courant d'argon, on introduit successivement à la pipette, le toluène et l'eau distillée préalablement dégazés sous azote, puis le catalyseur (complexe organométallique).

Deux variantes d'essais ont été réalisés :
- variante (a) avec 8 ml d'eau/8 ml de toluène/2 g de styrène d'une part et 1ml d'acétone/1 ml d'eau distillée/0,08 mmol de Rh/0,16 mmol de furylphosphine d'autre part
- variante(b) avec 2,5 ml d'eau/2,5 ml de toluène/1 g de styrène d'une part et 1ml de toluène/1 ml d'eau distillée/0,04 mmol de Rh/0,08 mmol de furylphosphine d'autre part.

On établit une pression de 10 bar (1 MPa) de monoxyde de carbone et de 10 bar (1 MPa) d'hydrogène. On chauffe en température (T°C) sous agitation magnétique vigoureuse pendant 18 h.

Après ventilation de l'excès de gaz, le mélange réactionnel est décanté en deux phases et la phase organique est analysée en chromatographie en phase gazeuse. Les résultats sont rassemblés dans le tableau 3 ci-après : rendement (Rendt) en aldéhyde et rapport aldéhyde ramifié/aldéhyde linéaire (Ar/Al).

**Tableau 3**

| **Exemple** | **Phosphine** **(exemple)** | **Précurseur** | **Variante** | **T°C** | **Rendt** | **Ar/Al** |
|---|---|---|---|---|---|---|
| Exemple 14 | préparée exemple 1 | [Rh(CO)₂Cl]₂ | (a) | 55°C | 32 % | 86/14 |
| Exemple 15 | préparée exemple 2 | [Rh(CO)₂acac] | (a) | 65°C | 100 % | 83/17 |
| Exemple 16 | préparée exemple 4 | [Rh(CO)₂acac] | (b) | 50°C | 69 % | 81/19 |

### EXEMPLES 17 à 26c : Utilisation de complexes métalliques avec différents ligands dans les réactions de couplage carbone-carbone en milieu aqueux (Réaction de Heck)

Dans un tube de Schlenk muni d'un barreau aimanté et sous atmosphère d'argon, on introduit
2 mmol (0,42 g) d'iodobenzène, 3 mmol ( 0,30g) d'acrylate d'éthyle, 3 mol ( 0,31 g) de triéthylamine et 3 ml d'acétonitrile.

On ajoute au milieu 0,05mmol (11,2 g) de Pd(O₂CCH₃)₂ puis un ligand phosphinique (0,15 mmol) sous forme dissoute dans 0,5 ml d'eau.

Le mélange est chauffé à une température déterminée sous vive agitation pour obtenir une homogénéisation du milieu.

En fin de réaction, la solution est filtrée puis transférée dans une ampoule pour être décantée.

Après plusieurs lavages à l'eau et à l'éther éthylique, on réalise une extraction des composés formés par de l'éther.

Après évaporation des solvants, une huile brune est recueillie.

Le taux de conversion de l'iodobenzène est déterminé par analyse par RMN du proton de l'huile brune recueillie.

La nature des ligands utilisés, la température et le temps de réaction sont indiqués dans le tableau 4 ci-dessous. Dans ce tableau est également précisé le taux de transformation de l'iodobenzène en cinnamate d'éthyle.
Les différents ligands utilisés sont :
- Ligand A : : 2,2'-phénylphosphino-bis(furyl-5-sulfinate de lithium)
- Ligand B : 2-(diphénylphosphino)-furyl-5-phosphonate de disodium
- Ligand C : 2-(diphénylphosphino)-furyl-5-carboxylate de sodium
- Ligand D : 2,2'-phénylphosphino-bis(furyl-5-carboxylate de lithium)
- Ligand E : métatriphenylphosphine trisulfonate de sodium (ligand de l'art antérieur)

**Tableau 4**

| **Exemple** | **17** | **18** | **19** | **20** | **21** | **22** | **23** | **24** | **25c** | **26c** |
|---|---|---|---|---|---|---|---|---|---|---|
| **Ligands** | A | | B | | C | | D | | E | |
| **T°C** | 22 | 80 | 40 | 70 | 40 | 80 | 40 | 80 | 40 | 70 |
| **Durée** **(Heure)** | 10 | 2,5 | 2 | 1 | 2 | 1 | 2 | 1 | 2 | 1 |
| **Taux de** **transformation de** **l'iodobenzène (%)** | 65 | 100 | 100 | 100 | 55 | 90 | 99 | 100 | 21 | 100 |

Les ligands conformes à l'invention permettent donc d'obtenir un taux de transformation de l'iodobenzène à des températures peu élevées et même à température ambiante.

### EXEMPLES 27 à 29c : Utilisation de complexes métalliques avec différents ligands dans la réaction d'isomérisation du 2-méthyl 3-butène nitrile (2M3RN) en 3-pentènenitrile (3PN)

Dans un tube de Schlenk muni d'un barreau aimanté et sous atmosphère d'argon, on introduit une 1,5 g de solution aqueuse d'un ligand hydrosoluble L avec une concentration exprimée en mmol/kg de ligand

On ajoute 5g de 2M3BN puis 40 à 45 mg de bis(1,5- cyclooctadiène)nickel(0) (Ni(COD)₂) pour obtenir dans le milieu un rapport molaire Ligand / Nickel égal à 4,5. Le mélange agité est porté à 90°C et maintenu à cette température pendant 3 heures. Après refroidissement, la masse réactionnelle est dissoute dans de l'acétone et analysée par Chromatographie en Phase Gazeuse pour doser les composés organiques présents. Les résultats obtenus avec plusieurs ligands dont deux conformes à l'invention sont rassemblés dans le tableau 5 ci-dessous.

**Tableau 5**

| **Exemple** | **27** | **28** | **29c** |
|---|---|---|---|
| **Ligands** | 2,2'-phénylphosphino -bis(furyl-5-sulfinate de lithium) | 2,2'-phénylphosphino-bis(furyl-5-phosphonate de disodium) | TPPTS Na₃ |
| **Concentration en** **ligand L** **(mmol/kg)** | 500 | 800 | 500 |
| **Rapport ligand /** **Ni** | 4,5 | 4,5 | 4,5 |
| **Taux de** **transformation du** **2M3BN (%)** | 20 | 87 | 68 |
| **Rendement en** **3PN (%)** | 73 | 92 | 87 |
| TPPTS : méta triphénylphosphine trisulfonée | | | |

Les ligands de l'invention permettent d'obtenir des taux de transformation du 2M3BN élevés avec une bonne sélectivité en 3PN.

Dans les tableaux 4 et 5 les exemples 25c, 26c et 29c sont des exemples comparatifs réalisés avec un ligand de l'art antérieur.

## Revendications

1. Furylphosphines, **caractérisées en ce qu'**elles sont hydrosolubles et répondent à la formule générale (I) : 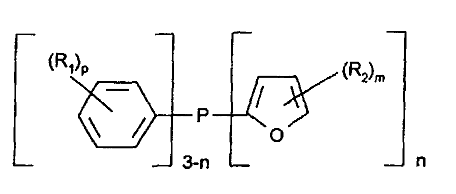 dans laquelle :
- n représente un nombre entier de 1 à 3
- au moins un radical R₂ représente un groupe hydrophile choisi dans le groupe comprenant :
- SO₂M, -SO₃M, -CO₂M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 12 atomes de carbone, les autres cations dérivés de métaux comprenant les dérivés métalliques obtenus avec des acides furylsulfiniques, des acides furylcarboxyliques, des acides furylsulfoniques ou des acides furylphosphoniques et qui sont solubles dans l'eau,
- N(R)₃X dans la formule duquel les symboles R, identiques ou différents, représentent un atome d'hydrogène ou un radical alkyle ayant de 1 à 12 atomes de carbone, X représente un anion organique ou minéral,
- OH,
- R₁ représente un groupe hydrophile selon la définition donnée pour R₂ ou un groupe alkyle ou alcoxy ayant 1 à 12 atomes de carbone, un atome d'halogène, un groupe nitrile ou un groupe halogénoalkyle ayant 1 à 12 atomes de carbone,
- m représente 1 ou 2,
- p représente un nombre entier de 0 à 3,
- lorsque m est égal à 2, un radical R₂ peut aussi représenter un groupe alkyle ou alcoxy ayant 1 à 12 atomes de carbone, un atome d'halogène, un groupe nitrile ou un groupe halogénoalkyle ayant 1 à 12 atomes de carbone.

2. Furylphosphines hydrosolubles selon la revendication 1, **caractérisées en ce qu'**elles répondent à la formule générale (I) dans laquelle :
- n représente un nombre entier de 1 à 3
- R₂ représente un groupe hydrophile choisi dans le groupe comprenant :
- SO₂M, -SO₃M, -CO₂M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux comprenant les dérivés métalliques obtenus avec des acides furylsulfiniques, des acides furylcarboxyliques, des acides furylsulfoniques ou des acides furylphosphoniques et qui sont solubles dans l'eau,
- m représente 1 ou 2
- R₁ représente un groupe hydrophile selon la définition donnée pour R₂ , ou un substituant :N(R)₃ X dans la formule duquel les symboles R, identiques ou différents, représentent un atome d'hydrogène ou un radical alkyle ayant de 1 à 4 atomes de carbone et X représente un anion organique ou minéral, ou un substituant -OH ou un substituant alkyle ou alcoxy ayant 1 à 4 atomes de carbone, un atome d'halogène, un groupe nitrile ou un groupe trifluoroalkyle,
- p représente un nombre entier de 0 à 2.

3. Procédé de préparation de furylphosphines hydrosolubles selon l'une des revendications 1 ou 2, **caractérisé en ce qu'**il consiste à réaliser une condensation d'un composé organolithien, dont la partie organique correspond à un composé de formule générale (I) ne comportant pas de substituants R₂ et qui est lié à l'atome de lithium par son ou ses cycles furyle avec un précurseur du groupe R2 choisi dans le groupe comprenant le dioxyde de soufre, le dioxyde de carbone, les chlorophosphates d'alkyle, les sulfonates de pyridine ou les sulfonates de trialkylamine

4. Complexes organométalliques, **caractérisés en ce qu'**ils comprennent au moins une furylphosphine hydrosoluble selon l'une des revendications 1 ou 2 et au moins un métal choisi parmi les métaux de transition des groupes 1b, 2b, 3b, 4b, 5b, 6b, 7b et 8 de la Classification périodique des éléments.

5. Complexes organométalliques selon la revendication 6, **caractérisés en ce qu'**ils comprennent au moins un métal choisi parmi le nickel, le cobalt, le fer, le ruthénium, le rhodium, le palladium, l'osmium, l'iridium, le platine, le cuivre, l'argent, l'or, le zinc, le cadmium, le mercure.

6. Procédé de préparation de complexes organométalliques selon l'une des revendications 4 ou 5, **caractérisé en ce que** l'on met en contact une solution d'un composé du métal choisi avec une solution aqueuse de la furylphosphine hydrosoluble de formule (I).

7. Procédé de préparation selon la revendication 6, **caractérisé en ce que** le composé du métal est dissous dans l'eau ou dans un solvant organique, ledit solvant organique pouvant lui-même être miscible ou non-miscible à l'eau.

8. Procédé de préparation selon l'une des revendication 6 ou 7, **caractérisé en ce que** le métal se trouve dans le composé mis en oeuvre, soit au degré d'oxydation qu'il aura dans le complexe organométallique, soit à un degré d'oxydation supérieur avec une réduction in situ.

9. Procédé selon la revendication 8, **caractérisé en ce que** quand le métal se trouve à un degré d'oxydation supérieur, une réduction in-situ de ce métal est réalisée.

10. Utilisation des complexes organométalliques selon l'une des revendications 4 ou 5 pour la catalyse des réactions d'hydroformylation et d'hydrocarbonylation des oléfines en présence de complexes du rhodium, d'hydrogénation des oléfines, des aldéhydes, des acides, des énamides et des composés nitroaromatiques en présence de complexes du ruthénium, du rhodium,
du platine ou du palladium, de télomérisation des diènes, d'isomérisation des oléfines, de dimérisation de l'éthylène ou de l'acrylonitrile, d'hydrocyanation des oléfines en présence de complexes du nickel, de synthèse du furanne en présence de complexes du ruthénium, de métathèse des oléfines en présence de complexes du ruthénium, la polymérisation des acrylates en présence de complexes du nickel.

## Claims

1. Furylphosphines, **characterized in that** they are water-soluble and correspond to the general formula (I): 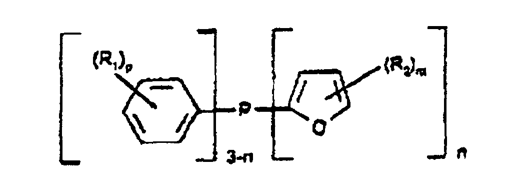 in which:
- n represents an integer from 1 to 3,
- at least one R₂ radical represents a hydrophilic group chosen from the group consisting of:
- SO₂M, -SO₃M, -CO₂M or -PO₃M, M representing an inorganic or organic cationic residue chosen from a proton, cations derived from alkali metals or alkaline-earth metals, ammonium cations -N(R)₄, in the formula of which the R symbols, which are identical or different, represent a hydrogen atom or an alkyl radical having from 1 to 12 carbon atoms, or other cations derived from metals comprising the metal derivatives obtained with furylsulphinic acids, furylcarboxylic acids, furylsulphonic acids or furylphosphonic acids and which are water-soluble,
- N(R)₃X, in the formula of which the R symbols, which are identical or different, represent a hydrogen atom or an alkyl radical having from 1 to 12 carbon atoms and X represents an organic or inorganic anion,
- OH,
- R₁ represents a hydrophilic group according to the definition given for R₂ or an alkyl or alkoxy group having 1 to 12 carbon atoms, a halogen atom, a nitrile group or a haloalkyl group having 1 to 12 carbon atoms,
- m represents 1 or 2,
- p represents an integer from 0 to 3,
- when m is equal to 2, an R₂ radical can also represent an alkyl or alkoxy group having 1 to 12 carbon atoms, a halogen atom, a nitrile group or a haloalkyl group having 1 to 12 carbon atoms.

2. Water-soluble furylphosphines according to Claim 1, **characterized in that** they correspond to the general formula (I) in which:
- n represents an integer from 1 to 3,
- R₂ represents a hydrophilic group chosen from the group consisting of:
-SO₂M, -SO₃M, -CO₂M or -PO₃M, M representing an inorganic or organic cationic residue chosen from a proton, cations derived from alkali metals or alkaline earth metals, ammonium cations -N(R)₄, in the formula of which the R symbols, which are identical or different, represent a hydrogen atom or an alkyl radical having from 1 to 4 carbon atoms, the other cations derived from metals comprising the metal derivatives obtained with furylsulphinic acids, furylcarboxylic acids, furylsulphonic acids or furylphosphonic acids and which are soluble in water,
- m represents 1 or 2,
- R₁ represents a hydrophilic group according to the definition given for R₂ or an -N(R)₃X substituent, in the formula of which the R symbols, which are identical or different, represent a hydrogen atom or an alkyl radical having from 1 to 4 carbon atoms and X represents an organic or inorganic anion, or an -OH substituent or an alkyl or alkoxy substituent having 1 to 4 carbon atoms, a halogen atom, a nitrile group or a trifluoroalkyl group,
- p represents an integer from 0 to 2.

3. Process for the preparation of water-soluble furylphosphines according to either of Claims 1 and 2, **characterized in that** it consists in condensing an organolithium compound, the organic part of which corresponds to a compound of general formula (I) which does not comprise R₂ substituents and which is bonded to the lithium atom via its furyl ring or rings, with a precursor from the group R2 chosen from the group comprising sulphur dioxide, carbon dioxide, alkyl chlorophosphates, pyridine sulphonates or trialkylamine sulphonates.

4. Organometallic complexes, **characterized in that** they comprise at least one water-soluble furylphosphine according to either of Claims 1 and 2 and at least one metal chosen from the transition metals from Groups 1b, 2b, 3b, 4b, 5b, 6b, 7b and 8 of the Periodic Classification of the Elements.

5. Organometallic complexes according to Claim 4, **characterized in that** they comprise at least one metal chosen from nickel, cobalt, iron, ruthenium, rhodium, palladium, osmium, iridium, platinum, copper, silver, gold, zinc, cadmium or mercury.

6. Process for the preparation of organometallic complexes according to either of Claims 4 and 5, **characterized in that** a solution of a compound of the chosen metal is brought into contact with an aqueous solution of the water-soluble furylphosphine of formula (I).

7. Preparation process according to Claim 6, **characterized in that** the metal compound is dissolved in water or in an organic solvent, it being possible for the said organic solvent itself to be miscible or immiscible with water.

8. Preparation process according to either of Claims 6 and 7, **characterized in that** the metal in the compound employed is either in the oxidation state which it will have in the organometallic complex or in a higher oxidation state with an in-situ reduction.

9. Process according to Claim 8, **characterized in that**, when the metal is in a higher oxidation state, an in-situ reduction of this metal is carried out.

10. Use of the organometallic complexes according to either of Claims 4 and 5 for the catalysis of reactions for the hydroformylation and hydrocarbonylation of olefins in the presence of rhodium complexes, for the hydrogenation of olefins, aldehydes, acids, enamides and nitroaromatic compounds in the presence of ruthenium, rhodium, platinum or palladium complexes, for the telomerization of dienes, for the isomerization of olefins, for the dimerization of ethylene or of acrylonitrile, for the hydrocyanation of olefins in the presence of nickel complexes, for the synthesis of furan in the presence of ruthenium complexes, for the metathesis of olefins in the presence of ruthenium complexes, and for the polymerization of acrylates in the presence of nickel complexes.

## Patentansprüche

1. Furylphosphine, **dadurch gekennzeichnet, daß** sie wasserlöslich sind und der folgenden allgemeinen Formel (I) 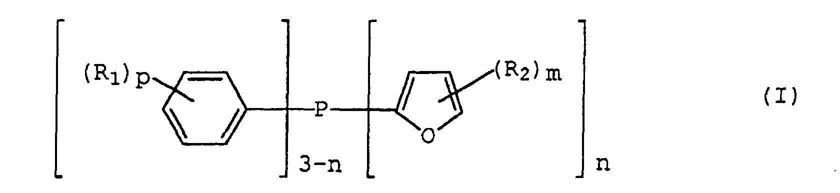 entsprechen, in der
- n eine ganze Zahl von 1 bis 3 darstellt,
- mindestens ein Rest R₂ eine hydrophile Gruppe bedeutet, gewählt aus der Gruppe, die umfaßt:
- -SO₂M, -SO₃M, -CO₂M, -PO₃M, worin M ein mineralischer oder organischer kationischer Rest ist, ausgewählt unter dem Proton, den von Alkalimetallen oder Erdalkalimetallen abgeleiteten Kationen, den Ammonium-Kationen -N(R)₄, worin die Symbole R, gleich oder verschieden, ein Wasserstoffatom oder einen Rest Alkyl mit 1 bis 12 Kohlenstoffatomen darstellen, wobei die anderen Kationen, die von Metallen abgeleitet sind, Metallderivate umfassen, die erhalten wurden mit Furylsulfinsäuren, Furylcarbonsäuren, Furylsulfonsäuren oder Furylphosphonsäuren und die in Wasser löslich sind,
- N(R)₃X, worin die Symbole R, gleich oder verschieden, ein Wasserstoffatom oder einen Rest Alkyl mit 1 bis 12 Kohlenstoffatomen darstellen und X ein mineralisches oder organisches Anion ist,
- OH,
- R₁ eine hydrophile Gruppe gemäß der bei R₂ gegebenen Definition ist oder eine Gruppe Alkyl oder Alkoxy mit 1 bis 12 Kohlenstoffatomen, ein Halogenatom, eine Gruppe Nitril oder eine Gruppe Halogenalkyl mit 1 bis 12 Kohlenstoffatomen bedeutet,
- m gleich 1 oder 2 ist,
- p eine ganze Zahl von 0 bis 3 ist,
- wenn m gleich 2 ist, ein Rest R₂ auch eine Gruppe Alkyl oder Alkoxy mit 1 bis 12 Kohlenstoffatomen, ein Halogenatom, eine Gruppe Nitril oder eine Gruppe Halogenalkyl mit 1 bis 12 Kohlenstoffatomen darstellen kann.

2. Wasserlösliche Furylphosphine nach Anspruch 1, **dadurch gekennzeichnet, daß** sie der allgemeinen Formel (I) entsprechen, in der
- n eine ganze Zahl von 1 bis 3 darstellt,
- R₂ eine hydrophile Gruppe bedeutet, gewählt aus der Gruppe, die umfaßt:
-SO₂M, -SO₃M, -CO₂M, -PO₃M, worin M ein mineralischer oder organischer kationischer Rest ist, ausgewählt unter dem Proton, den von Alkalimetallen oder Erdalkalimetallen abgeleiteten Kationen, den Ammonium-Kationen -N(R)₄, worin die Symbole R, gleich oder verschieden, ein Wasserstoffatom oder einen Rest Alkyl mit 1 bis 4 Kohlenstoffatomen darstellen, wobei die anderen Kationen, die von Metallen abgeleitet sind, Metallderivate umfassen, die erhalten wurden mit Furylsulfinsäuren, Furylcarbonsäuren, Furylsulfonsäuren oder Furylphosphonsäuren und die in Wasser löslich sind,
- m gleich 1 oder 2 ist,
- R₁ eine hydrophile Gruppe gemäß der bei R₂ gegebenen Definition ist oder einen Substituenten -N(R)₃X bedeutet, worin die Symbole R, gleich oder verschieden, ein Wasserstoffatom oder einen Rest Alkyl mit 1 bis 4 Kohlenstoffatomen darstellen und X ein mineralisches oder organisches Anion oder einen Substituenten -OH oder einen Substituenten Alkyl oder Alkoxy mit 1 bis 4 Kohlenstoffatomen, ein Halogenatom, eine Gruppe Nitril oder eine Gruppe Trifluoralkyl bedeutet,
- p eine ganze Zahl von 0 bis 2 ist.

3. Verfahren zur Herstellung von wasserlöslichen Furylphosphinen nach einem der Ansprüche 1 oder 2, **dadurch gekennzeichnet, daß** es darin besteht, eine Kondensation einer Organolithiumverbindung, deren organischer Teil einer Verbindung der allgemeinen Formel (I) entspricht, die keine Substituenten R₂ umfaßt, und die an das Lithiumatom durch ihren oder ihre Furylring(e) gebunden ist, mit einem Vorläufer der Gruppe R₂ zu realisieren, gewählt aus der Schwefeldioxid, Kohlendioxid, die Alkylchlorphosphate, die Pyridinsulfonate oder die Trialkylamin-sulfonate umfassenden Gruppe.

4. Organometallkomplexe, **dadurch gekennzeichnet, daß** sie mindestens ein wasserlösliches Furylphosphin nach einem der Ansprüche 1 oder 2 und mindestens ein Metall umfassen, ausgewählt unter den Übergangsmetallen der Gruppen 1b, 2b, 3b, 4b, 5b, 6b, 7b und 8 des Periodensystems der Elemente.

5. Organometallkomplexe nach Anspruch 4, **dadurch gekennzeichnet, daß** sie mindestens ein Metall umfassen, ausgewählt unter Nickel, Cobalt, Eisen, Ruthenium, Rhodium, Palladium, Osmium, Iridium, Platin, Kupfer, Silber, Gold, Zink, Cadmium und Quecksilber.

6. Verfahren zur Herstellung von Organometallkomplexen nach einem der Ansprüche 4 oder 5, **dadurch gekennzeichnet, daß** man eine Lösung einer ausgewählten Metallverbindung mit einer wäßrigen Lösung des wasserlöslichen Furylphosphins der Formel (I) in Kontakt bringt.

7. Verfahren zur Herstellung nach Anspruch 6, **dadurch gekennzeichnet, daß** die Metallverbindung in Wasser oder in einem organischen Lösungsmittel gelöst wird, wobei das genannte organische Lösungsmittel selbst mit Wasser mischbar oder nicht mischbar sein kann.

8. Verfahren zur Herstellung nach einem der Ansprüche 6 oder 7, **dadurch gekennzeichnet, daß** das Metall sich in der eingesetzten Verbindung entweder in einem Oxidationsgrad befindet, den es in dem Organometallkomplex haben wird, oder in einem höheren oxidationsgrad, mit einer Reduktion in situ.

9. Verfahren nach Anspruch 8, **dadurch gekennzeichnet, daß** in dem Fall, wo sich das Metall in einem höheren Oxidationsgrad befindet, eine Reduktion dieses Metalls in situ durchgeführt wird.

10. Verwendung der Organometallkomplexe nach einem der Ansprüche 4 oder 5 für die Katalyse von Reaktionen der Hydroformylierung und Hydrocarbonylierung von Olefinen in Anwesenheit von Komplexen des Rhodiums, der Hydrierung von Olefinen, Aldehyden, Säuren, Enamiden und nitroaromatischen Verbindungen in Anwesenheit von Komplexen des Rutheniums, Rhodiums, Platins oder Palladiums, der Telomerisierung von Dienen, der Isomerisierung von Olefinen, der Dimerisierung von Ethylen oder Acrylnitril, der Hydrocyanierung von Olefinen in Anwesenheit von Komplexen des Nickels, der Furan-Synthese in Anwesenheit von Komplexen des Rutheniums, der Metathese von Olefinen in Anwesenheit von Komplexen des Rutheniums, der Polymerisation von Acrylaten in Anwesenheit von Komplexen des Nickels.
